(19) Europäisches Patentamt
European Patent Office
Office européen des brevets

(11) **EP 4 124 289 A1**

(12) **EUROPEAN PATENT APPLICATION**

(43) Date of publication:
**01.02.2023 Bulletin 2023/05**

(21) Application number: **21188758.3**

(22) Date of filing: **30.07.2021**

(51) International Patent Classification (IPC):
*A61B 5/0205* (2006.01)   *A61B 5/024* (2006.01)
*A61B 5/11* (2006.01)   *A61B 5/00* (2006.01)

(52) Cooperative Patent Classification (CPC):
**A61B 5/0205; A61B 5/024; A61B 5/1116;**
**A61B 5/721; A61B 5/7282**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DENISSEN, Adrianus Johannes Maria**
**5656 AG Eindhoven (NL)**

• **ARTS, Lukas**
**5656 AG Eindhoven (NL)**
• **WESTERINK, Joanne Henriëtte Desirée Monique**
**5656 AG Eindhoven (NL)**
• **Van DEN BROEK, Egidius Leon**
**5656 AG Eindhoven (NL)**

(74) Representative: **Philips Intellectual Property &**
**Standards**
**High Tech Campus 52**
**5656 AG Eindhoven (NL)**

(54) **DEVICE, SYSTEM AND METHOD FOR DETERMINING HEALTH INFORMATION RELATED TO THE CARDIOVASCULAR SYSTEM OF A SUBJECT**

(57) The present invention relates to a device, system and method for determining health information related to the cardiovascular system of a subject. To improve the accuracy of the determined health information the device comprises a HR input (31) configured to obtain a time-dependent heart rate, HR, related signal representing or allowing to derive the subject's heart rate over time; an event selection input (32) configured to obtain an event selection signal allowing to detect a movement event of the subject; a processing unit (33) configured to determine the health information related to the cardiovascular system of a subject from the obtained HR related signal and the obtained event selection signal; and an output (34) configured to output the determined health information. The processing unit (33) is configured to detect movement events of the subject based on the event selection signal, extract time segments of the HR related signal, a time segment covering a period including the respective movement event, combine the extracted time segments to compute an aggregated HR response signal, and extract one or more features from the aggregated HR response signal to determine the health information related to the cardiovascular system of the subject.

FIG.2

EP 4 124 289 A1

## Description

FIELD OF THE INVENTION

The present invention relates to a device, system and method for determining health information related to the cardiovascular system of a subject, e.g. for determining a so-called HeartAge for a subject as an indicator of the health of the subject's cardiovascular system.

BACKGROUND OF THE INVENTION

Nowadays, people keep track of sleep, nutrition, overall health, and numerous other aspects of their lives with the use of apps and wearables. Cardiovascular monitoring based e.g. on the Electrocardiogram (ECG) is currently successfully used for the identification of diseases, stress-analysis, prognosis, biometric identification, etc. However, ECG requires the placement of multiple sensors at specific positions, which makes it inefficient as wearable. Photoplethysmography (PPG) sensors solve this problem by measuring heart rate (HR) and heart rate variability (HRV) with pulse oximeters that emit light on the skin and measure the intensity of the reflection. This intensity is dependent on blood flow beneath the skin that is regulated by the cardiac cycle. Measuring the periodicity in the reflection then results in estimations of HR and HRV. The fact that this measurement can be done at different portions of the body, e.g. the wrist, fingertips or earlobes, makes PPG sensors convenient in daily life and popular for integration in wearable devices like smartwatches. In addition, it is found that PPG sensors can measure changes of the physical and even mental state of the body by only monitoring the heart. In addition to that, remote PPG has been found to be able to detect vital signs, such as HR, respiration rate, SpO2, from a camera signal of a skin portion of the subject.

Obtaining a clean PPG signal is the biggest challenge in wearables as it contains unpredictable artefacts. Therefore, most research and applications are performed in controlled environments with minimal physical movement. PPG signal windows that are contaminated by movement-based noise are either ignored or filtered out.

Despite the enormous potential of the use of PPG in monitoring applications, PPG still has a lot of issues regarding noise and unreliable measurements. These artefacts prevent the use of PPG as a clinical monitoring device.

SUMMARY OF THE INVENTION

It is an object of the present invention to provide a device, system and method for determining health information related to the cardiovascular system of a subject, which are preferably operating in an unobtrusive manner and can optionally be used during rest or sleep of the subject.

In a first aspect of the present invention a device for determining health information related to the cardiovascular system of a subject is presented, the device comprising:

a HR input configured to obtain a time-dependent heart rate, HR, related signal representing or allowing to derive the subject's heart rate over time;
an event selection input configured to obtain an event selection signal allowing to detect a movement event of the subject;
a processing unit configured to determine the health information related to the cardiovascular system of a subject from the obtained HR related signal and the obtained event selection signal; and
an output configured to output the determined health information, wherein the processing unit is configured to

- detect movement events of the subject based on the event selection signal,
- extract time segments of the HR related signal, a time segment covering a period including the respective movement event,
- combine the extracted time segments to compute an aggregated HR response signal, and
- extract one or more features from the aggregated HR response signal to determine the health information related to the cardiovascular system of the subject.

In a further aspect of the present invention a system for determining health information related to the cardiovascular system of a subject is presented, the system comprising:

a HR sensor configured to sense a time-dependent heart rate, HR, related signal representing or allowing to derive the subject's heart rate over time;
a selection signal sensor configured to sense an event selection signal allowing to detect a movement event of the subject;
a device as disclosed herein for determining the health information related to the cardiovascular system of a subject from the sensed HR related signal and the sensed event selection signal; and
an output interface configured to issue the determined health information.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to focus on time segments of a HR related signal (e.g. a PPG signal) that are "contaminated" with movement-based noise. Thus, in contrast to known methods for patient monitoring, movement events of the subject are detected and the corresponding time segments of the HR related signal that cover periods including the respective movement event are used for the subsequent processing and determining information about the subject's cardiovascular system.

The present invention thus presents a solution that can overcome the technical limitations of known monitoring devices, systems and method and that allows determining a subject's one's cardiovascular vitality unobtrusively, e.g. during the night, by monitoring HR reactivity after movement or in other situations, e.g. in daytime, where movements of the subject affect HR (e.g. when dismounting the own car).

As this cardiovascular vitality (i.e. the determined health information) is often correlated to the subject's actual age, this new physiological measure may be called "HeartAge", which can be related to the subject's biological age. As such, it can serve as a powerful, single value for "HeartAge": the "age" of the subject's cardiovascular system in the sense of indication of the adaptability/flexibility of the subject's cardiovascular system. By monitoring the subject's "HeartAge" continuously without the presence of a caregiver (e.g. a nurse or doctor), it may be possible to determine and/or signal the risk on various cardiovascular issues in a very early stage. As such, the present invention may save lives and may be a huge addition to the near future healthcare. Generally, the invention may be used to determine other pieces of health information related to the subject's cardiovascular system, and present them in terms of e.g. weight ("HeartWeight"), BMI ("HeartBMI"), vitality ("HeartVitality"), happiness ("HeartHappiness"), stress level ("HeartStress"), etc.

In this context, "detecting" of movement events does not only mean to actually measure or identify movement events, but also means to predict or estimate movement events. For instance, it is possible that a time segment of the related HR signal is first extracted after a movement event, and then this time segment is extended back in time so that it does include the movement event.

According to a preferred embodiment the processing unit is configured to align the extracted time segments before combining based on the event selection signal or an obtained alignment signal that indicates time information of a movement event within the respective time segment. This further improves the accuracy of the determination of the health information. Generally, the event selection signal may be used for alignment as well, i.e., the event selection signal may not only be used for selecting movement events, but also for aligning extracted time segments, but a separate alignment signal may be used as well. For instance, an accelerometer (ACC) signal from e.g. a body-worn ACC sensor may be used as alignment signal and event selection signal. In other embodiments, the ACC signal may be used as alignment signal and a non-ACC signal (e.g. a weight or pressure sensor signal sensing movements or posture changes of the subject) may be used as event selection signal. In still other embodiments the ACC signal may be used as event selection signal and a non-ACC signal may be used as alignment signal.

The processing unit may advantageously be configured to detect a movement event by detecting a movement of the subject that affects the subject's HR, in particular a recurring movement of the subject or a movement that occurs more often, such as a change of posture of the subject or a turning movement of the subject. One or more sensor signals of one or more body-worn sensors (e.g. an ACC sensor) and/or external sensors (e.g. a camera or a pressure sensor) may be used for this purpose.

Preferably, the processing unit may be configured to detect a movement event by detecting one or more movement features of movement of the subject, said movement features including movement intensity above an intensity threshold, movement pattern, movement speed, movement direction, and movement distance.

In a preferred embodiment the processing unit is configured to detect, among the selected time segments, one or more unwanted time segments during which the HR related signal is distorted or during which the HR is unaffected despite detection of a movement event, in particular based on one or more characteristics of the respective selected time segments, and discard the detected unwanted time segments for the combining of the extracted time segments. This further improves the accuracy of the determination of the health information. Besides time segments in which HR is distorted, also time segments in which HR is unaffected (despite the detection of a movement event) are removed and not used in the further processing to determine the desired health information. The HR may e.g. be unaffected by movements of the subject if movements are wrongfully detected, e.g. due to signal noise or other distorting causes. By removing such time segments where HR remains (substantially or completely) stationary, based on the main assumption that movement causes a change in HR, a correction for these errors is made a posteriori.

In another embodiment the processing unit is configured to extract a time segment of the HR related signal by determining the characteristic point in time, at which a particular characteristic of a detected event appears, and to select the time segment around said characteristic point in time to span a first time period before said characteristic point in time and a second time period after said characteristic point in time. The characteristic point in time may e.g. be the time at which a change of position or posture of the subject actually happened. This characteristic point in time can be recognized e.g. in the event selection signal and/or the HR related signal. A period around (or only after) said characteristic point in time may then be chosen from the HR related signal as time segment. The period may be predetermined and fixed or may be individually set or chosen by the user or the processing unit. Reasonably values for this period may be in the range of 10 to 240 seconds, in particular in the range of 30 to 120 seconds. The actual value may depend on the application of the invention and may be different from those values, i.e. the actual value may generally be smaller or larger than the minimum and maximum values mentioned.

The processing unit may further be configured to discard, for the combining of the extracted time segments, time periods in the respective selected time segments, at which the detected movement event happens and only use time periods in the respective selected time segments before and/or after the detected movement event. This contributes to further improvement of the accuracy since it has been found that at the moment of the movement event the HR related signal may be noisy or distorted, i.e., the motion may lead to artefacts in the HR related signal, so that this portion of the HR related signal should be discarded and only portion(s) of the HR related signal directly before and/or after the movement event should be used as time segment.

Generally, different features of the aggregated HR response signal can be used to determine the desired health information. In an embodiment the processing unit may be configured to extract, as one or more features from the aggregated HR response signal, one or more of

- the difference between maximum value and minimum value of the aggregated HR response signal,
- the difference between maximum value and start value of the aggregated HR response signal,
- the difference between end value and start value of the aggregated HR response signal,
- the time difference between appearance of minimum value and maximum value of the aggregated HR response signal,
- the time difference between appearance of start value and end value of the aggregated HR response signal,
- the maximum gradient of aggregated HR response signal, and
- the duration and/or speed of an increase of the aggregated HR response signal.

The features generally have the goal of describing the aggregated heart rate response in one number. Therefore, one or more of these features (or even additional features, such as area under the aggregated HR response signal, amount of heart beats before returning to normal, etc.) may be used that serve this purpose.

Preferably, the processing unit may be configured to determine the health information related to the cardiovascular system of the subject by use of a predetermined function, in particular a linear or non-linear approximation applying one or more predetermined scalar parameters. The scalar parameters may be predetermined, e.g. for various types of subjects (e.g. based on age, gender, size, weight, health status, fitness level, etc.) or may be individually set or determined for the particular subject under examination.

In an embodiment the processing unit may be configured to determine the scalar parameters from one or more relationships between age of the subject and the respective one or more features from the aggregated HR response signal. Such a determination is preferably made in advance based on empirical data showing or allowing to derive the relationship between age and one or more features of the aggregated HR response signal that shall be used in the actual determination of the subject's health information.

Generally, various kinds of signal can be used as HR related signal. Preferably, one of a contact or remote photoplethysmography (PPG) signal, a HR signal, an ECG signal, a ballistocardiogram, a tonometric signal, or a holographic laser Doppler imaging signal is used in practical implementations.

For the event selection signal different options exist as well, e.g. based on which sensor can be used or which signal is available in the particular application scenario. In practical implementations, the event selection signal may be one of a movement signal indicating movement of the subject's body, an accelerometer signal, a muscle activity signal, a brain activity signal, a pressure sensor signal, a weight sensor signal, a CAN (Controller Area Network) bus signal (or other bus signal) of a vehicle, and a camera signal.

For the alignment signal there are different options as well, again which sensor can be used or which signal is available in the particular application scenario. In practical implementations, the alignment signal may be one of a movement signal indicating movement of the subject's body, an accelerometer signal, a muscle activity signal, a brain activity signal, a pressure sensor signal, a weight sensor signal, a CAN bus signal (or other bus signal) of a vehicle, and a camera signal.

For combining (e.g. averaging) the multiple time segments of the HR related signal into a final aggregated HR response signal several options exist.

In a preferred embodiment, the processing unit is configured to combine the extracted time segments by first converting the extracted time segments to a signal representing frequency over time (e.g., using a wavelet transform) and then aggregating these signals into an aggregated HR response signal, i.e., the HR related signal is first converted into a signal describing its frequencies with respect to time and, subsequently, the extracted time segments are combined by aggregating the extracted time segments. In another embodiment the processing unit is configured to combine the aggregated time segments by first transforming the extracted time segments into time-frequency matrices, combining the matrices, in particular on a pixel-by-pixel basis, and then taking a maximum ridgeline to obtain an aggregated HR response signal, i.e., the corresponding time-frequency matrices are combined and then a maximum ridgeline is taken as the aggregated HR response signal.

BRIEF DESCRIPTION OF THE DRAWINGS

These and other aspects of the invention will be apparent from and elucidated with reference to the embodiment(s) described hereinafter. In the following drawings

   Fig. 1 shows a schematic diagram of an embodiment of a system according to the present invention.
   Fig. 2 shows a schematic diagram of an embodiment of a device according to the present invention.
   Fig. 3 shows a schematic diagram of a first embodiment of a method according to the present invention.
   Fig. 4 shows a schematic diagram of a second embodiment of a method according to the present invention.
   Fig. 5 shows a diagram of a time-frequency matrix.
   Fig. 6 shows a diagram illustrating the summation and averaging of time segments of the HR related signal.
   Fig. 7 shows a diagram illustrating that the aggregated HR response signal remains stable over multiple nights.
   Fig. 8 shows a diagram illustrating the relationship between intensity of aggregated HR response signal and age of the subject.

DETAILED DESCRIPTION OF THE EMBODIMENTS

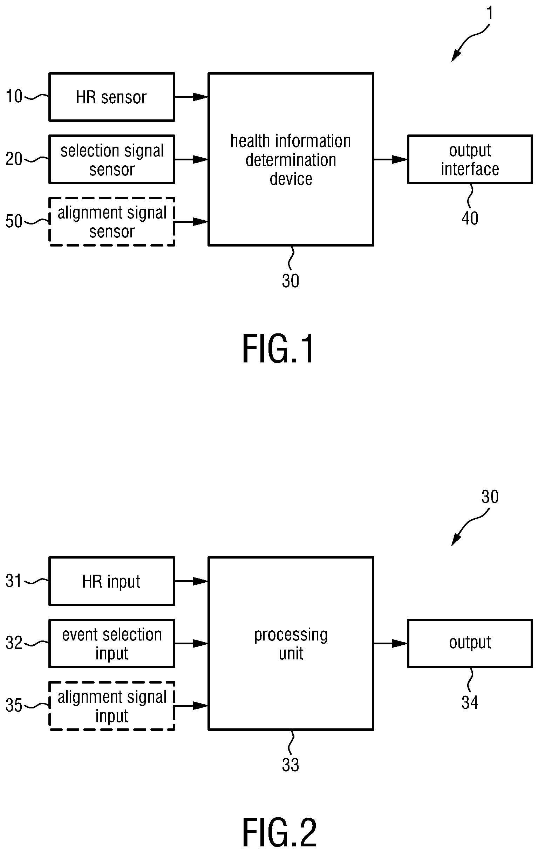

Fig. 1 shows a schematic diagram of an embodiment of a system 1 for determining health information related to the cardiovascular system of a subject according to the present invention. The subject may generally be any human being, but the invention may find particular application in monitoring of patients, elder people, people with critical cardiovascular system, sportspersons, etc.
The system 1 comprises a heart rate (HR) sensor 10 configured to sense (i.e. acquire or measure) a time-dependent HR related signal representing or allowing to derive the subject's HR over time. The HR sensor 10 may be a wearable sensor, such as a contact photoplethysmography (PPG) sensor, a HR rate sensor, an ECG sensor. In other embodiments the HR sensor may be an external sensor, such a remote PPG sensor, a ballistocardiogram sensor, a tonometry sensor, or a holographic laser Doppler imaging sensor. The HR related signal may thus directly show the subject's HR over time, e.g. in case of a contact PPG signal or a HR rate sensor, or may allow to derive the subject's HR, e.g. in case of a remote PPG signal or a ballistocardiogram signal.
The system 1 further comprises a selection signal sensor 20 configured to sense an event selection signal allowing to detect a movement event of the subject. The selection signal sensor 20 may be any wearable or external sensor that provides a signal from which it can be detected if the subject performs a movement or is going to perform a movement, particularly a movement that affects the subject's HR. The signal may directly show the movement or may allow to derive that there is or will be a movement of the subject. A movement event may thus be any event where the subject changes position and/or posture, in particular to a certain extent or in a certain way so that the subject's HR shows a - potentially temporary - change as a consequence of the subject's movement. In preferred embodiments the selection signal sensor 20 may be a movement sensor providing a movement signal indicating movement of the subject's body, an accelerometer, a muscle activity sensor, a brain activity sensor, a pressure sensor, a weight sensor, a CAN bus providing a CAN bus signal of a vehicle, or a camera.
The system 1 further comprises a device 30 configured to determine the health information related to the cardiovascular system of a subject from the sensed HR related signal and the sensed event selection signal. Details of the device 30 will be described below. The device 30 may e.g. be implemented as or in a processor or computer, in software and/or hardware.
The system 1 further comprises an output interface 40 configured to issue the determined health information. The output interface 40 may generally be any means that outputs the determined health information in visual or audible form, e.g. in text form, as image or diagram, as sound or spoken words, etc. For instance, the output interface 40 may be a display, a loudspeaker, a touchscreen, a computer monitor, the screen of a smartphone or tablet, etc.
Optionally, the system 1 may further comprise an alignment signal sensor 50 configured to sense an alignment signal allowing to align time segments extracted from the HR related signal before combining them. For this alignment the event selection signal may be used (in which case the alignment signal sensor 50 may be omitted). Otherwise, a separate alignment signal may be obtained that indicates time information of a movement event within the respective time segment so that the time segments can be aligned in time based on this time information.
Fig. 2 shows a schematic diagram of an embodiment of a device 30 for determining health information related to the cardiovascular system of a subject according to the present invention.
The device 30 comprises a HR input 31 configured to obtain the time-dependent HR related signal and an event selection input 32 configured to obtain the event selection signal. Optionally, an additional alignment signal input 35 is provided to obtain the alignment signal if available. The HR input 31 and the event selection input 32 (and the optional alignment signal input 35) may be directly coupled or connected to the HR sensor 10 and the event signal sensor 20 (and the optional alignment signal sensor 50), respectively, or may obtain (i.e. retrieve or receive) these signals from a storage, buffer, network, or bus, etc. The inputs 31, 32 and 35 may thus e.g. be (wired or wireless) communication interfaces or data interfaces, such as a Bluetooth interface, WiFi interface, LAN interface, HDMI interface, direct cable connect, or any other suitable interface allowing signal transfer to the device 30.

The device 30 further comprises a processing unit 33 configured to determine the health information related to the cardiovascular system of a subject from the obtained HR related signal and the obtained event selection signal. The processing unit 33 may be any kind of means configured to process the signals and determine the health information there from. It may be implemented in software and/or hardware, e.g. as a programmed processor or computer or app on a user device such as a smartphone, smartwatch, tablet, laptop, PC, workstation, etc.

The device 30 further comprises an output 34 configured to output the determined health information. The output 34 may generally be any interface that provides the determined health information, e.g. transmits it to another device or provides it for retrieval by another device (e.g. a smartphone, computer, tablet, etc.). It may thus generally be any (wired or wireless) communication or data interface.

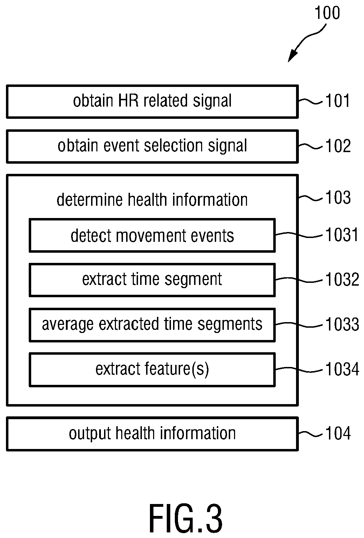

Fig. 3 shows a schematic diagram of a first embodiment of a method 100 for determining health information related to the cardiovascular system of a subject according to the present invention. The steps of the method may be carried out by the device, wherein the main steps of the method are carried out by the processing unit. The method may be implemented as computer program running on a computer or processor.

In a first step 101 the time-dependent HR related signal is obtained, e.g. retrieved or received from the HR sensor 10. In a second step 102, carried out before, after or in parallel to step 101, the event selection signal is obtained, e.g. retrieved or received from the event signal sensor 20. Subsequently, in step 103 the health information related to the cardiovascular system of a subject is determined from the obtained HR related signal and the obtained event selection signal. Finally, in step 104 the determined health information is output.

Step 103 of determining the health information comprises a first step 1031 of detecting movement events of the subject based on the event selection signal. For the respective movement events, in step 1032 time segments are extracted from the HR related signal, a time segment covering a period including the respective movement event. In step 1033 the extracted time segments are combined (e.g. averaged) to compute an aggregated HR response signal. From the aggregated HR response signal one or more features are extracted in step 1034 to determine the health information related to the cardiovascular system of the subject using the extracted one or more features.

In another embodiment the device 30 of the present invention may comprise three modules:

    i) a recording module (including or commonly representing the inputs 31, 32 and 35) that records a PPG signal (as HR related signal), a selection signal, and an alignment signal;
    ii) a processing module (representing the processing unit 33) which combines these signals and extracts characteristic Aggregated Heart Rate Responses (AHRRs) and determines health information there from; and
    iii) a feedback module (representing the output 34) that summarizes and reports the results. In an embodiment the processing module (or processing unit) extracts AHRRs by

        i) using bandpass filters to preprocess the PPG signal and selection signal,
        ii) selecting relevant movement events using the selection signal,
        iii) denoising and extracting heart rate curves from PPG signal during these movement events,
        iv) detecting and removing heart rate curves that are severely distorted, and
        v) aligning and averaging all remaining heart rate curves using the alignment signal, and, thereby, obtaining the AHRR.

The resulting AHRR is shown to be stable per person. It is used to estimate the person's health information such as the person's HeartAge. These steps will be explained in more detail below.

The feedback module preferably summarizes and reports all results over a specified period of time. Feedback can be given to the user or to a caregiver, such as a doctor or nurse. Feedback can be provided in several forms (e.g., text and visualizations).

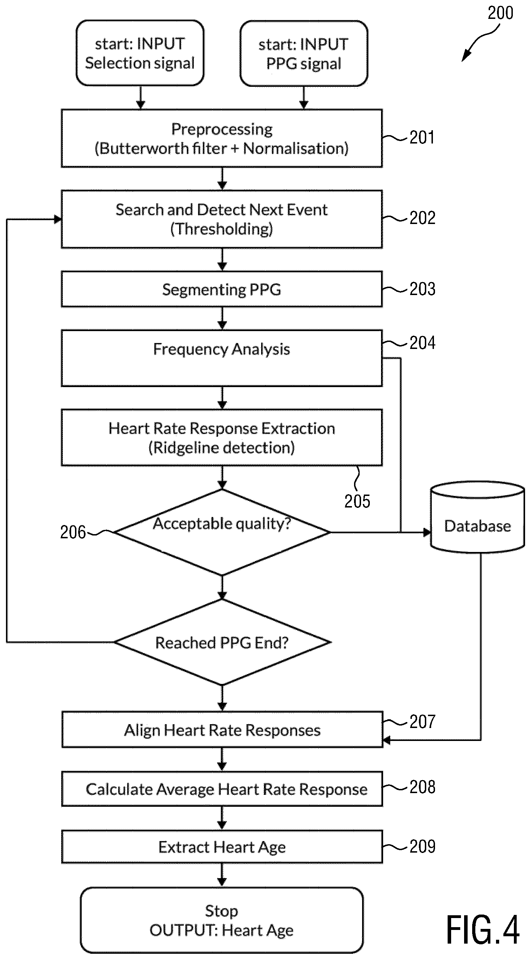

In an embodiment the algorithm to extract the AHRR takes a PPG signal (as HR related signal) and a selection signal as input. Suitable signals can be gathered during sleep as heart rate is lowered and more stable, when compared to daytime. People make between 20 and 80 small (turning) movements during their sleep. These give rise to a typical temporary increase in heart rate (possibly related to the so-called cardiovascular orthostatic reflex). Acceleration signals can be used as selection signal to detect the start of a turn movement. Fig. 4 shows a schematic diagram of a second embodiment of a method 200 according to the present invention making use such an algorithm. This embodiment particularly performs a series of steps in order to denoise the signals and determine the AHRR.

First, preprocessing is performed in step 201 on the first derivative of the PPG signal, as an example using a band-pass 3rd order Butterworth filter with cutoff frequencies of 0.5 - 2 Hz. The first derivative is used to cancel out baseline wandering and make peak detection easier. Cutoff frequencies of 0.5 - 2 Hz correspond to an expected HR range of 30 - 120 BPM (Beats Per Minute) and allow the extraction of the HR's fundamental frequency. Furthermore, both the selection signal and PPG signal may be normalized to a signal-dependent fixed range, using a sliding time window around the sample that spans [-0.5, 0.5] seconds. The signals may be zero-padded at the beginning and end to ensure correct normalization of the first and last samples. To handle large spikes and negative parts in the signal, an x/(max - min) normalization may be used to guarantee a positive fixed signal range.

Second, the turning events (as one example of "movement events") during sleep are detected (step 202) and the PPG signal is segmented into multiple windows of interest (step 203). These event windows (the "time segments") are defined as distinctive intervals in time during which the respective event takes place. A movement event can generally be anything that triggers a sudden HR response, i.e., a change of the HR. Events may be selected to have a definite start, a definite end, and a considerable resting period between them. A possibility is to define the start and end of a turning event window based on a temporary above-a-certain-threshold increase in the accelerometer signal: For each event, the end of the accelerometer increase may be used. The start of the event window may be defined as a first time period before the end of the accelerometer increase (e.g. in the range of 10 to 90 before it, e.g. 40 seconds before it), and the end of the event window may be defined as another second time period after the end of the accelerometer increase (e.g. in the range of 10 to 90 after it, e.g. 60 seconds after it). In this way, the accelerometer signal (used as selection signal) may also function as alignment signal to align the different event windows before further processing. Other characteristic points (different from the end of the accelerometer increase) may be used as well for defining the event window and the first and second periods of the event window mentioned above.

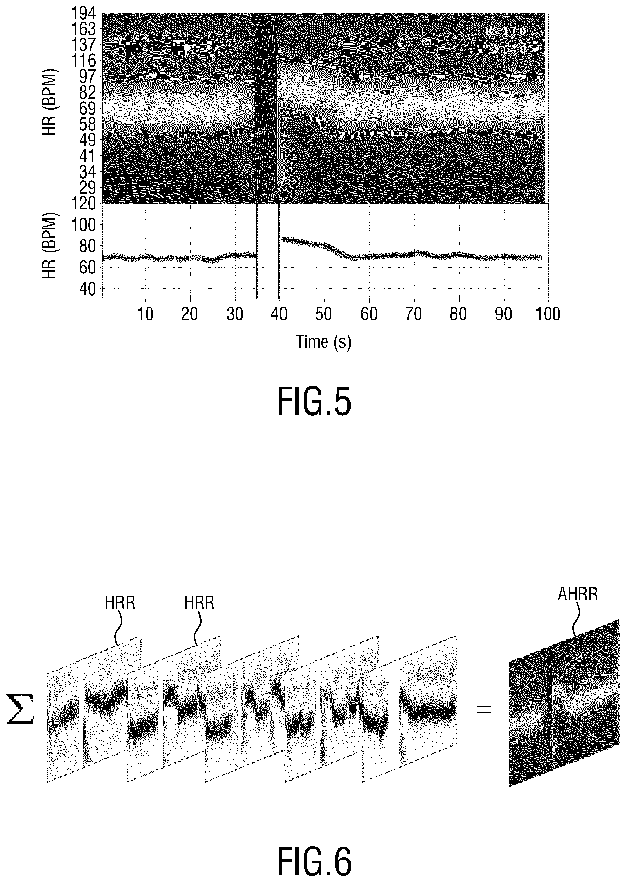

Third, the PPG signal during the windows of interest is converted (step 304) to a Time-Frequency Matrix (e.g. a Continuous Wavelet Transformation Matrix) as shown in Fig. 5, in which each column describes the frequency spectrum at time t. The PPG signal exactly during the event is preferably ignored as it is often too distorted at the moment of the event. Then, this matrix is compressed, e.g. by a (samplefreq x 1) max-pool filter. The resulting matrix has a resolution of one column per second and is called the Heart Rate Response (HRR). The vertical position of the maximal values of these columns corresponds to a frequency. These frequencies may be extracted to get the Maximal Ridgeline (MR) that describes the HRR in BPM as shown in the bottom diagram of Fig. 5.

As the PPG signal is very sensitive to distortion, maximal pixel values in the time-frequency matrix sometimes correspond to frequencies caused by noise instead of frequencies caused by the HR. Therefore, anomalies may be detected by subtracting a filtered MR, e.g. a 9s median filtered MR, from the original MR in step 205. The residue is compared to a threshold to find large deviations indicating outlier behavior. An asymmetric threshold that prefers high HR outliers may be used. Change in HR tends to be asymptotic near minimum and maximum HR. A resting HR is therefore expected to have a higher possible increase than decrease. A residue larger than 30 percent or smaller than 10 percent of the previous HR may be omitted from further processing. Resulting gaps shorter than the average event duration may be linearly interpolated.

This embodiment enables removing false measurements (outliers). The number of outliers may give a hint about HRR quality. However, it is not the only measure used. The process of removing HRRs that are too distorted may comprise two 2 steps: In a first step, characteristics of the HRR signal are measured and in a second step it is chosen whether to remove a HRR curve based on its characteristics. For instance, in the first step several features are extracted from the HRR curve. These features describe the HRR's form, stability, variance, number of bad measurements, etc. Basically, the features describe all necessary information for a human to make the decision about HRR quality himself. However, a human may be asked, but shall preferably not be asked to decide. Instead, an algorithm may be trained to make this decision automatically. For this purpose, in the second step, after extracting the features, an algorithm is trained to decide whether a curve has decent quality the HRR curve is too distorted to be of any use. Too distorted means that a meaningful HRR cannot be extracted. As explained above, a Random Forest algorithm may be used to do this binary decision task. Such a Random Forest algorithm can be trained with a plurality (e.g. several hundreds) of manually labeled HRRs. This labelling and training of the algorithm may be done once for many applications or specifically for every application, i.e., it may be seen as a kind of calibration. These HRRs were labeled by hand as being of good or bad quality. Based on these HRR curves and all extracted features, the Random Forest algorithm learns to detect whether a curve is of good or bad quality. However, other classifiers may be used instead of the Random Forest algorithm.

Next, in step 206 the interpolated HRR is analyzed, and temporal and morphological features are calculated that serve as a feature space for a discrimination algorithm. The discrimination algorithm (e.g. a random forest classifier containing e.g. 100 trees with a maximum depth of e.g. 10 levels) has learned to detect and remove HRRs that do not describe an actual heart rate response (e.g. are too flat) or that are too distorted for further processing.

Finally, the remaining HRR signals of a plurality of (preferably all) event windows are normalized, aligned (step 207) and then combined (e.g. averaged) together (step 208), emphasizing reoccurring patterns, and thereby obtaining the AHRR. This is illustrated in Fig. 6 showing multiple HRRs and the resulting AHRR. Because the event windows are then aligned, combining separate event windows allows revealing reoccurring HR behavior and cancel random errors and fluctuations. As indicated above, HRRs may be aligned on the event's ending time based on the selection signal the main interest is directed to HR behavior following the event. As a result, it has been found that AHRR characteristics are very stable per person (intraclass correlation = 0.7-0.8) over several nights. Features are then extracted from these AHRRs obtained e.g. for several nights to determine the subject's health information related to the subject's cardiovascular system, e.g. the vitality and condition of the artery system, also referred to as the HeartAge herein (step 209).

The use of context alignment in the denoising process allows for detailed HR behavior analysis with a resolution that has never been seen before in wearable applications. This increase in resolution introduces new possibilities to continuously monitor vitality and HeartAge unobtrusively at night. A more detailed example (the HeartAge example) will be described below.

As mentioned above, many different signals may be used as selection signal and/or alignment signal (e.g., torso acceleration and leg muscle activity). Data may be obtained in full or partial overnight unattended home recordings. But also in other situations data may be obtained, e.g., in an office where the person repeatedly changes posture between sitting and standing, when a person is doing sports with changes in movements or posture, when a person is leaving a vehicle (i.e., stands up from the seat and leaves the vehicle), etc. The fact that multiple sensors may be used and that AHRRs were stable within each person shows that the determination and use of AHRR is a versatile, robust, accurate and stable new measuring technique.

The alignment of the various HRRs or time segments of the HR related signal can be also done in other ways, e.g. by using cross-correlation. Then, the HRRs or the time segments may themselves function as the alignment signal. For instance, two HRRs may be correlated with each other for a whole range of delays, and the delay that produces the highest correlation is selected. This happens for all pairs of two HRR curves (e.g. all pairs of two HRR curves in the night), and each HRR signal is then shifted by the amount of its average delay, before the procedure is resumed to calculate the AHRR from the shifted HRR curves. This method will also enable to identify different types of HRR curves from the heights of the correlations between individual HHR curves: those HRR curves that correlate well with each other (for some delay) belong to the same cluster. Then, for each cluster a specific AHHR can be constructed. Most likely, the different clusters will relate to different types of turning movements during the night.

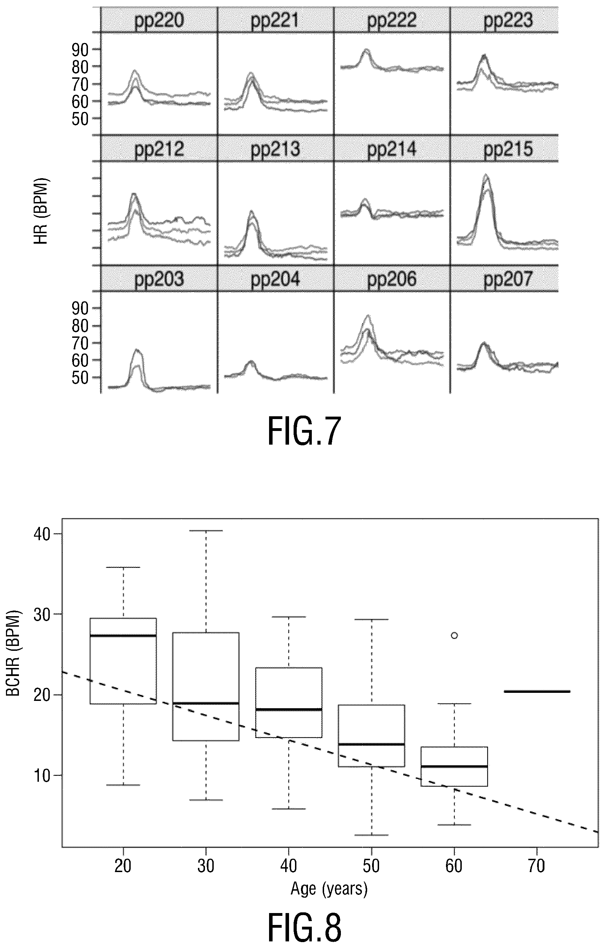

Generally, many movements, e.g. each turn during sleep, generate a HRR that is specific for one's body. Averaging these HRRs gives an even more stable AHRR per night that is characteristic for a specific body and does not change significantly between nights, as illustrated in Fig. 7 showing multiple AHRRs taken at different nights (each plot shows 3 AHRR curves for a single subject taken during 3 nights). Using the AHHR signal as calculated above, the "HeartAge" may be calculated as follows:

$$HeartAge = a + b*(AHRRincrease),$$

with a and b being scalars that are preferably determined empirically, and AHRR being the Aggregated Heart Rate Responses as described above. The AHHR increase may be determined as the maximum HR in the AHHR signal minus the AHHR at t=0. This AHRRincrease might be related to the so-called orthostatic reflex: the increase in HR when someone is changing to a more upright posture (see e.g. G. Cybulski and W. Niewiadomski, "Influence of age on the immediate heart rate response to the active orthostatic test," J Physiol Pharmacol, vol. 54, no. 1, pp. 65-80, 2003).

The embodiment described above calculates HeartAge from AHRRincrease by means of a linear approximation. This is the simplest way to do it, and generally it suits the available data well. Given enough available data for the relevant situation (e.g. standing up from the car or from a chair), any other function F(x) (x being a feature of AHRR and F being the desired health information such as HeartAge) may generally be fitted to the data, for instance a second order function $F(x)=a+bx+cx^2$ Thus, in certain applications a different function may be applied to the available data set, especially when it is collected for a different situation (like leaving the car).

Fig. 8 shows the relationship between a subject's AHRRincrease and actual age: On average the higher the subject's age is, the lower the subject's AHRRincrease. This overall population relationship then serves to enable an estimation of the linear dependence for HeartAge and the parameters a and b. Thus, the HeartAge of a person can be considered the age at which the overall population has the same AHRRincrease as his/hers is.

The fact that the extraction of AHRR can be taken at home without the assistance of a doctor creates new possibilities to optimize physical training or medication. Effortless longitudinal monitoring of vitality and HeartAge is thereby enabled.

In Fig. 8 the relationship between age and AHRRincrease is depicted as determined from a particular data set. As can be seen in the graph, it follows a linear formula AHRRincrease = C1 + C2*age with C1 = 25.5 bpm and C2 = -0.275 bpm/year. The determination of HeartAge from AHRRincrease just follows the inverse function: HeartAge = (AHRRincrease - C1)/C2 = a + b*AHRRincrease.. In other words, the scalars a and b described above in the approach using a linear approximation may be determined from the linear equation of the fitted line shown in Fig. 8 such that b= 1/C2= 3.64 year/bpm, and a = -C1/C2= 92.7 years. Other scalars using in other approximations for determining HeartAge (or other health information) may be determined using the same or a similar approach based on a relationship between age and one or more features of the AHRR determined in advance for a larger population based on empirical data. Further, such scalar may be determined in advance for certain types of subjects based on typical parameters of patients, such as age, gender, size, weight, fitness level, health status, medication, etc.

Generally, to detect the HeartAge one or more features may be extracted from the AHRR. In the embodiments described above, AHRRincrease is used as feature, but one or more other features may be used instead or in addition. In the case of turning events during sleep, the posture changes somewhat during the turning, but then returns to horizontal. As a consequence, it is expected that the HR before turning will be roughly the same as the HR (well) after turning, with a temporary increase in HR during turning and shortly afterwards. For stepping out of a car, the situation is different: there the posture is sitting before the posture change and standing after the posture change, and it is known that the HR while standing is higher than while sitting. It is hence expected to see a lasting increase in HR, but maybe with an overshoot during the process of standing up. In this case, both the lasting increase in HR as well as the height of the overshoot are parameters that can indicate health status or HeartAge.

Other features of the AHRR may be used as well, such as

- the difference between maximum value and minimum value of the aggregated HR response signal,
- the difference between maximum value and start value of the aggregated HR response signal,
- the difference between end value and start value of the aggregated HR response signal,
- the time difference between appearance of minimum value and maximum value of the aggregated HR response signal,
- the time difference between appearance of start value and end value of the aggregated HR response signal,
- the maximum gradient of aggregated HR response signal, and
- the duration and/or speed of an increase of the aggregated HR response signal.

Real-time implementation of the present invention is possible since only a small window of interest around a movement event must generally be analyzed. Movement events must be separated by at least the duration of one window of interest. As the algorithm only consists of inexpensive computations, this analysis can be done right after the window of interest of the event ends. Consecutive HRRs or extracted time segments of the HR related signal can be added to a temporary AHRR which is divided by the number of events in the end to get the final AHRR. This processing pipeline allows real-time calculation of AHRR in remote applications where computing power and battery resources are limited.

With the introduction of smartwatches, the use of wearable devices grew exponentially in the last few years and is expected to experience this growth even more in the coming years. The fact that wearable wristband devices are often connected to smartphones via Bluetooth of WiFi allows for remote processing of PPG data (or, in general, HR related signals) on smartphones. As one of the embodiments of AHRR estimation only uses a PPG and acceleration sensor, AHRR estimation could ideally be done using a smartwatch and a user-friendly smartphone app. Due to the vast inter-connectivity and frequent usage of smartphones, use-cases of AHRR in daily life are endless.

One potential application of the present invention is measuring HeartAge or other health information via a wristband (or any other wearable) that a vehicle driver or a vehicle passenger is using. Each time when the driver leaves the car, the sensors in chair and door are registering this activity, and therefore can function as a selection signal. When leaving the care, the driver changes from a seated posture towards a standing posture, and this triggers a true orthostatic reflex in their cardiovascular system: the heart rate increases to accommodate the standing position (and in doing so, often temporarily overshoots). These heart rate changes are measured by the PPG sensor in the wristband. Using the seat & door CAN signal as a selection signal, a range of moments can be selected from the PPG data, in which the driver changes from a seated to an upright position, and the present invention allows the calculation of AHRR and HeartAge for this particular situation. The same holds for the combination of passenger seat & door and the wristband of the person sitting there.

Thus, in such an embodiment the CAN bus signal of a car that the driver door opens can be used as a selection signal, indicating that in the next minute or so, it is expected that the driver change from a sitting to a standing posture (i.e., the CAN bus signal can be used to predict a future movement event and estimate the time of the movement event), and as a consequence, to assume that the HR of the driver will increase. The ACC signal may then be used as an indication when exactly the driver stands up, i.e. as an alignment signal. The advantage of such a combination is, that the selection signal can be stricter in correct selection of the appropriate movement events, while the alignment is better at determining (and aligning) the exact timing of multiple movement events and the related time segments of the HR related signal during these movement events.

In another embodiment an ACC signal may be used as selection signal and a non-ACC may be used as alignment signal. For instance, in the case where an activity sensor (e.g. an ACC sensor) sensor is put on the chest of a subject and an EMG sensor (sensing muscle tension; non-ACC sensor) on the triceps muscle of the subject, the activity sensor detects that the subject starts moving and that the subject's chest goes upward. This allows selecting a HR trace that captures the fact that the chest is rising (and an HR reaction is expected). The EMG sensor on the triceps muscle indicates the exact moment when the subject starts use the muscles to stand up so that the EMG sensor signal can be used for alignment.

Generally, different pieces of health information may be determined using the present invention. One example is the HeartAge described above. Another example is the weight of a person as another indicator of the person's health, just like age generally is. The main information to get by use of the present invention is information about the flexibility/adaptivity of the cardiovascular system. The information about the flexibility/adaptivity of the cardiovascular system may then be back-translated to something the layperson might understand better, such as his age, weight or BMI.

To determined weight, HeartWeight may be constructed, similar to HeartAge, giving an indication of the adaptivity/flexibility of the heart by comparing one or more features of the AHRR, such as the AHHRincrease, with that of other persons with various weights. Hence, the HeartWeight might tell a person that despite the person's overweight (the person e.g. weighing above 100 kg), heart responses are similar to that of a person who is much leaner, i.e., persons HeartWeight might only be 80 kg. This would actually be the same health information as for HeartAge, namely information about the flexibility/adaptivity of the cardiovascular system. It would only be presented in a different way.

The same applies for other pieces of heart information, such as BMI ("HeartBMI"), vitality ("HeartVitality"), happiness ("HeartHappiness"), stress level ("HeartStress"), etc.

The present invention enables unobtrusive, ambulatory, stable measurement of people's cardiovascular vitality. Using e.g. wearable PPG measurements during sleep, people's "HeartAge" can be determined. The invention may exploit different signals, e.g. a wearable's accelerometer signal, to identify people's movements, e.g. turns during sleep. Subsequently, it alienates the HR changes around these moments using a time-frequency conversion method (e.g. continuous wavelet transformation). As such, the present invention takes the inverse perspective of normal practice: it utilizes the an event detection signal, e.g. the wearable's accelerometer signal, to extract specific signal windows of a HR related signal, which would otherwise be omitted as movement-based noise.

The present invention may find particular application in home monitoring of health and wellbeing as well as sleep and respiratory care. Further, the present invention provides an easy means of diagnosis. Another application area is passenger wellness in automotive applications.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining health information related to the cardiovascular system of a subject, the device comprising:

   a HR input (31) configured to obtain a time-dependent heart rate, HR, related signal representing or allowing to derive the subject's heart rate over time;
   an event selection input (32) configured to obtain an event selection signal allowing to detect a movement event of the subject;
   a processing unit (33) configured to determine the health information related to the cardiovascular system of a subject from the obtained HR related signal and the obtained event selection signal; and
   an output (34) configured to output the determined health information,

   wherein the processing unit (33) is configured to

   - detect movement events of the subject based on the event selection signal,
- extract time segments of the HR related signal, a time segment covering a period including the respective movement event,
- combine the extracted time segments to compute an aggregated HR response signal, and
- extract one or more features from the aggregated HR response signal to determine the health information related to the cardiovascular system of the subject.

2.  Device as claimed in claim 1,
    wherein the processing unit (33) is configured to align the extracted time segments before combining based on the event selection signal or an obtained alignment signal that indicates time information of a movement event within the respective time segment.

3.  Device as claimed in any one of the preceding claims,
    wherein the processing unit (33) is configured to detect a movement event by detecting a movement of the subject that affects the subject's HR, in particular a recurring movement of the subject, such as a change of posture of the subject or a turning movement of the subject.

4.  Device as claimed in any one of the preceding claims,
    wherein the processing unit (33) is configured to detect a movement event by detecting one or more movement features of movement of the subject, said movement features including movement intensity above an intensity threshold, movement pattern, movement speed, movement direction, and movement distance.

5.  Device as claimed in any one of the preceding claims,
    wherein the processing unit (33) is configured to detect, among the selected time segments, one or more unwanted time segments during which the HR related signal is distorted or during which the HR is unaffected despite detection of a movement event, in particular based on one or more characteristics of the respective selected time segments, and discard the detected unwanted time segments for the combining of the extracted time segments.

6.  Device as claimed in any one of the preceding claims,
    wherein the processing unit (33) is configured to extract a time segment of the HR related signal by determining the characteristic point in time, at which a particular characteristic of a detected event appears, and to select the time segment around said characteristic point in time to span a first time period before said characteristic point in time and a second time period after said characteristic point in time.

7.  Device as claimed in any one of the preceding claims,
    wherein the processing unit (33) is configured to discard, for the combining of the extracted time segments, time periods in the respective selected time segments, at which the detected movement event happens and only use time periods in the respective selected time segments before and/or after the detected movement event.

8.  Device as claimed in any one of the preceding claims,
    wherein the processing unit (33) is configured to extract, as one or more features from the aggregated HR response signal, one or more of

    - the difference between maximum value and minimum value of the aggregated HR response signal,
    - the difference between maximum value and start value of the aggregated HR response signal,
    - the difference between end value and start value of the aggregated HR response signal,
    - the time difference between appearance of minimum value and maximum value of the aggregated HR response signal,
    - the time difference between appearance of start value and end value of the aggregated HR response signal,
    - the maximum gradient of aggregated HR response signal, and
    - the duration and/or speed of an increase of the aggregated HR response signal.

9.  Device as claimed in any one of the preceding claims,
    wherein the processing unit (33) is configured to determine the health information related to the cardiovascular system of the subject by use of a predetermined function, in particular a linear or non-linear approximation applying one or more predetermined scalar parameters.

10. Device as claimed in claim 9,
    wherein the processing unit (33) is configured to determine the scalar parameters from one or more relationships between age of the subject and the respective one or more features from the aggregated HR response signal.

11. Device as claimed in any one of the preceding claims,
    wherein the HR related signal is one of a contact or remote photoplethysmography, PPG, signal, a HR signal, an ECG signal, a ballistocardiogram, a tonometric signal, or a holographic laser Doppler imaging signal, and/or

wherein the event selection signal is one of a movement signal indicating movement of the subject's body, an accelerometer signal, a muscle activity signal, a brain activity signal, a pressure sensor signal, a weight sensor signal, a CAN bus signal of a vehicle, and a camera signal.

12. Device as claimed in any one of the preceding claims,
wherein the processing unit (33) is configured to combine the extracted time segments by

i) first converting the extracted time segments to a signal representing frequency over time and then aggregating these signals into an aggregated HR response signal, or
ii) first transforming the extracted time segments into time-frequency matrices, combining the matrices, in particular on a pixel-by-pixel basis, and then taking a maximum ridgeline to obtain an aggregated HR response signal.

13. System for determining health information related to the cardiovascular system of a subject, the system comprising:

a HR sensor (10) configured to sense a time-dependent heart rate, HR, related signal representing or allowing to derive the subject's heart rate over time;
a selection signal sensor (20) configured to sense an event selection signal allowing to detect a movement event of the subject;
a device (30) as claimed in any one of the preceding claims for determining the health information related to the cardiovascular system of a subject from the sensed HR related signal and the sensed event selection signal; and
an output interface (40) configured to issue the determined health information.

14. Method for determining health information related to the cardiovascular system of a subject, the method comprising:

obtaining a time-dependent heart rate, HR, related signal representing or allowing to derive the subject's heart rate over time;
obtaining an event selection signal allowing to detect a movement event of the subject;
determining the health information related to the cardiovascular system of a subject from the obtained HR related signal and the obtained event selection signal; and
outputting the determined health information,

wherein the health information is determined by

- detecting movement events of the subject based on the event selection signal,
- extracting time segments of the HR related signal, a time segment covering a period including the respective movement event,
- combining the extracted time segments to compute an aggregated HR response signal, and
- extracting one or more features from the aggregated HR response signal to determine the health information related to the cardiovascular system of the subject.

15. Computer program comprising program code means for causing a computer to carry out the steps of the method as claimed in claim 14 when said computer program is carried out on the computer.

10 — | HR sensor |

20 — | selection signal sensor |

50 — | alignment signal sensor |

| health information determination device |

30

| output interface |

40

1

## FIG.1

31 — | HR input |

32 — | event selection input |

35 — | alignment signal input |

| processing unit |

33

| output |

34

30

## FIG.2

100

obtain HR related signal ~101

obtain event selection signal ~102

determine health information ~103

detect movement events ~1031

extract time segment ~1032

average extracted time segments ~1033

extract feature(s) ~1034

output health information ~104

FIG.3

200

start: INPUT
Selection signal

start: INPUT
PPG signal

Preprocessing
(Butterworth filter + Normalisation) ~201

Search and Detect Next Event
(Thresholding) ~202

Segmenting PPG ~203

Frequency Analysis ~204

Heart Rate Response Extraction
(Ridgeline detection)

205

206~ Acceptable quality?

Database

Reached PPG End?

Align Heart Rate Responses ~207

Calculate Average Heart Rate Response ~208

Extract Heart Age ~209

Stop
OUTPUT: Heart Age

FIG.4

FIG.5

FIG.6

FIG.7

FIG.8

Europäisches
Patentamt

European
Patent Office

Office européen
des brevets

# EUROPEAN SEARCH REPORT

Application Number

EP 21 18 8758

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/249951 A1 (BONOMI ALBERTO GIOVANNI [NL] ET AL) 6 September 2018 (2018-09-06) * paragraphs [0008], [0017], [0051], [0057], [0059], [0078]; figures 1,3 * ----- | 1-15 | INV. A61B5/0205 A61B5/024 A61B5/11 A61B5/00 |
| A | US 2019/183430 A1 (ALPHONSE RICKY [US] ET AL) 20 June 2019 (2019-06-20) * paragraphs [0011], [0012], [0045], [0142] * ----- | 1-15 | |
| A | US 2019/290147 A1 (PERSEN KEN [US] ET AL) 26 September 2019 (2019-09-26) * paragraphs [0003], [0007], [0009], [0056] * ----- | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 13 January 2022 | Chau, Thoi Dai |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 18 8758

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2022

| Patent document cited in search report | | Publication date | Patent family member(s) | | Publication date |
|---|---|---|---|---|---|
| US 2018249951 | A1 | 06-09-2018 | CN | 107405110 A | 28-11-2017 |
| | | | EP | 3258846 A1 | 27-12-2017 |
| | | | JP | 6496087 B2 | 03-04-2019 |
| | | | JP | 2018538081 A | 27-12-2018 |
| | | | RU | 2017128430 A | 11-02-2019 |
| | | | US | 2018249951 A1 | 06-09-2018 |
| | | | WO | 2017108640 A1 | 29-06-2017 |
| US 2019183430 | A1 | 20-06-2019 | NONE | | |
| US 2019290147 | A1 | 26-09-2019 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. CYBULSKI ; W. NIEWIADOMSKI.** Influence of age on the immediate heart rate response to the active orthostatic test. *J Physiol Pharmacol,* 2003, vol. 54 (1), 65-80